# EUROPEAN PATENT SPECIFICATION

(11) **EP 1 917 959 B1**
(45) Date of publication and mention of the grant of the patent: **19.01.2011**
(21) Application number: 07460015.6
(22) Date of filing: 03.07.2007
(51) Int. Cl.: A61K 31/194, A61P 31/00

(54) **New medical use of alfa-ketoglutarate**
Neue medizinische Verwendung von Alpha-Ketoglutarat
Nouvel usage médical de l'alpha-cétoglutarate

(30) Priority: 03.07.2006 PL 38010306
(43) Date of publication of application: 07.05.2008
(73) Proprietor: Kruszewska, Danuta, 02-765 Warszawa (PL)
(72) Inventor: Kruszewska, Danuta, 02-765 Warszawa (PL)
(74) Representative: Malewska, Ewa

(56) References cited:
- WO-A-2005/123056
- SCHLEGEL LAURENT ET AL: "Bacterial dissemination and metabolic changes in rats induced by endotoxemia following intestinal E. coli overgrowth are reduced by ornithine alpha-ketoglutarate administration" JOURNAL OF NUTRITION, vol. 130, no. 12, December 2000 (2000-12), pages 2897-2902, XP002472372 ISSN: 0022-3166
- DATABASE WPI Section Ch, Week 1974 Derwent Publications Ltd., London, GB; Class D13, AN 1974-44619V XP002472373 "Antiseptic agent contg. a ketoglutaric acid - esp. useful in the food ind" & JP 49 019855 B (UENO PHARMACEUTICAL) 1974

## Description

This invention relates to a new medical application of alpha-ketoglutarate for manufacturing of therapeutic and prophylactic preparatioms for use in human medicine and in veterinary medicine in vertebrates, including mammals, birds, fish and amphibian.

### Background of the invention

Alpha-ketoglutarates - salts of alpha-ketoglutaric acid

Alpha-ketoglutarate occurs in living organisms as an endogenous molecule.

Salts of alpha-ketoglutaric acid have been known for at least 60 years, i.e. since the Krebs cycle was discovered. Endogenous alpha-ketoglutaric acid salts play in humans and in animals a fundamental role, together with the salts of oxaloacetic and pyruvic acid, in the citric acid cycle. As a result of reproducible reactions there are synthesised fatty acids, sterols, cholesterol (with involvement of citrate), porphiryn, heme, chlorophyll (activity of succinyl-CoA), glutamate, amino acids, nucleotide bases (activity of alpha-ketoglutaric acid salts).

Alpha-ketoglutaric acid anion play a key role in metabolism, mainly in aerobic organisms. Alpha-ketoglurate is produced in a process of oxidative decarboxylation involving cellular enzyme of isocitrate dehydrogenase, and also in another metabolic pathway, by an oxidative deamination of glutamate catalyzed by glutamate dehydrogenase.

Alpha ketoglutarate - being an intermediate compound in the basic life cycle, namely the tricarboxylic acid cycle, i.e. the Krebs cycle, is a subject of intracellular permanent transformations and it is present in small concentrations in peripheral blood in an anion form due to its full metabolisation in the organism.

At the same time, alpha-ketoglutarate plays within the organism a role of a natural scavenger - a decontaminant, by transporting nitrogen by means of transamination in effect of a transfer of amino groups originating from catabolised amino acids. This process occurs in the liver and it is called the ornithine cycle or urea cycle.

As alpha-ketoglutarate may undergo transamination together with glutamine, during such process a neurotransmitter named glutamate is formed. In the presence of B6 vitamin, glutamate may be decarboxylated while producing a compound referred to as GABA (gamma-aminobutyric acid) which is an inhibitor of the neurotransmitter (that blocks the neurotransmitter).

It has also been shown that alpha-ketoglutarate enzymes participate in the removal of free radicals - very toxic compounds - not completely metabolised products, from the organism.

Besides, one of alpha-ketoglutarate-dependent oxygenases is a molecular oxygen sensor, i.e. an indicator of oxygen level in the environment.

Alpha-ketoglutarate may also be introduced into an organism through known routes of administration, for instance: orally, through inhalation, intravenously or via other routes.

The everyday diet of both humans and animals does not comprise alpha-ketoglutarate.

On the market, in particular in America, there are commercially available dietary supplements containing salts of alpha-ketoglutaric acid, mainly salts of arginine, pyridoxine, ornithine, creatine, histidine and citrulline, as ready-made products for humans and for household animals. Also available are sodium, potassium and calcium salts of alpha-ketoglutaric acid.

In the dietary supplements register drawn up by the National Nutritional Foods Association (NNFA), alpha-ketogluteric acid itself and alpha-ketogluteric acid combined with pyridoxine (vitamin B6) was listed in the group of biochemical products even before September 15, 1994. Such a long presence on the market results in a large number of dietary supplements containing these chemical compounds. The main reason why in all the products available comprising derivatives or salts of alpha-ketogluteric acid is that alpha-ketoglutarate - as an intermediate compound in the Krebs cycle - is one of the substances responsible for cellular respiration and therefore it is believed to improve quality of life.

The most numerous group among the products discussed here are the products comprising L-arginine in combination with alpha-ketoglutarate. These products, as declared by manufacturers, facilitate the maintenance of energy during and after physical activities, enhance nitrogen oxide synthesis, besides - in combination with nitrogen oxide, they increase the level of this oxide in the organism and enhance transportation of nutrients, as well as enhance metabolism in muscles. In combination with other substances, thay also increases the level of energy and enhance amino acid metabolism.

The next largest group of the marketed products comprises ornithine in combination with alpha-ketoglutaric acid. In such a form alpha-ketoglutarate not only increases energy production, but also protects muscles against decomposition of branched amino acids in order to generate glutamine, the energy boosting molecule. Besides, it is a compound which enhances secretion of growth hormone and optimises muscle metabolism. It increases the activity of insulin and polyamines in a safe way. It also enhances neurotransmission thus helping to maintain a good mental status of the organism, supports endurance of muscles maximising athletic effects, influences the organism's fat burning capability, increases libido, has a beneficial influence on immunological functions and reduces oxygen stress.

Further group of food preparations used as dietary supplements comprises pyridoxine and pyridoxyl that are combined with alpha-ketoglutarate. Components of these products enhance metabolic activity within a cell, balance out the organism's efforts to produce energy and protect the liver.

On the market there are also products comprising creatine in combination with alpha-ketoglutarate. They act as glutamine precursors and participate in synthesis of proteins.

Other, non-specified alpha-ketogluteric acid salts stimulate fat reduction in the organism and are requisite of integrity of muscle tissue.

Alpha-ketogluteric acid itself is on the other hand a component of a preparation exhibiting a natural detoxifying function. It is recommended for use in chronic fatigue and in metabolic deficiencies often diagnosed by amino acids analysis. Administration of this type of preparation results in increase of stamina and boosts energy. An interesting product of this group is a calcium and magnesium salt of alpha-ketoglutaric acid. Due to the fact that alpha-ketoglutaric acid belongs to a group of strong organic acids, it irritates oesophagus and stomach when administered orally. Use of calcium and magnesium allows production of a buffered bi-component alpha-ketoglutaric acid compound that does not cause the unpleasant feeling of excessive acidity.

Numerous patents and patent applications show that alpha-ketoglutarate may be administered in metabolic brain dysfunction, in disorders of the nervous system, blood circulation, skeletal muscle system, in order to strengthen cell mitochondrion functions.

Drinks comprising alpha-ketoglutaric acid salts are also available and are intended to supply energy to the organism, in particular before, during and after physical activity. Such a drink - as a source of energy - may be administered also in states of fast and long-lasting demand for energy in humans and other mammals.

Furthermore, alpha-ketoglutarate may be used as a non-steroid, anabolic product increasing muscle mass without transforming muscles into fat. The effect of such a dietary supplement is similar to the effect obtained with synthetic anabolic steroids, however without the unfavourable side effects.

Orally may also be administered a supplement comprising solouble in fats tiamin, lipoic acid, creatine derivatives and L-arginine with alpha-ketoglutarate. This dietary supplement is intended to lower blood glucose levels and to maintain low glucose levels in the course of treatment of diabetic neuropathy, as well as to improve blood circulation and muscle efficiency.

Alpha-ketoglutaric acid salts were found to have an interesting application as agents aiming to reduce nitrogen emission in humans and animals and to maintain protein synthesis, and also in food microbiology.

Use of alpha-ketoglutaric acid and its salts as component(s) of an antiseptic agent intended for in food industry has been disclosed. See: DATABASE WPI, Section Ch, Week 1974, Derwent Publications Ltd., London, GB; Class D13, AN 1974-44619V XP002472373 "Antiseptic agent contg. a ketoglutaric acid - esp. useful in the food ind." And JP 49 01 9855 B (UENO PHARMACEUTICAL), 1974. The antibacterial activity of alpha-ketoglutaric acid and a number of other organic acids such as ascorbic and propionic acids, or their salts - illustrated by experimental results for *Bacillus subtilis,* consists of preventing growth of bacteria rather than killing the microorganisms. In this context it should be noted, however, that salt (NaCl) alone - present in the food products tested, has the same antibacterial activity. Finally, the concentration of alpha-ketoglutaric acid or its salts tested in the experiments ranges from 0.05 to 1.0 % in food products preserved with the agent, is unsuitable for use in living organisms.

In the food industry the salts are used as a component improving and enhancing aroma and taste of fermentation products (e.g. vinaigrette) and dairy products, *inter alia* cheese. Alpha-ketoglutaric acid salts influence lactic acid bacteria fermentation thus changing metabolism of amino acids, levels of catabolites and activity of aminotransferases. In practice, it leads to shortening of the cheese ripening period by accelerating formation of those compounds which guarantee high commercial quality of food products. See: Williams AG, Noble J, Banks JM., The effect of alpha-ketoglutaric acid on amino acid utilization by nonstarter Lactobacillus spp. isolated from Cheddar cheese. Lett. Appl. Microbiol. 2004;38:289-295.

Numerous patents and patent applications relate to use of alpha-ketoglutarate as a pharmaceutical preparation or as a component of a pharmaceutical preparation.

A use of alpha-ketoglutaric acid, glutamine, glutamic acid and salts thereof as well as amides and di- and tripeptides as pharmaceutical preparations for treatment and prophylaxis of arthrosis, rheumatoid arthritis and cartilage damage due to inflammation and other reasons is known from the publication WO 2007/058612.

Publication WO 2005/123056 discloses a use of alpha-ketoglutaric acid, glutamine, glutamic acid and pharmaceutically acceptable salts thereof, amides and di- and tripeptides as a pharmaceutical preparation, as food and animal feed additive in the treatment and prophylaxis of excessive plasma levels of at least one of the following parameters: cholesterol, LDL, glycerides. The preparation may also be used to raise HDL level.

EP 0 922 459 discloses that alpha-ketogluteric acid together with D-galactose and ornithine and such alpha-ketogluteric acid salts as sodium, potassium, magnesium, zinc and calcium salts in a defined dosage, in the form of tablets, powders, infusions, syrups may serve to raise amino acids profile in blood, in particular in patients under metabolic stress situations. The disclosed preparation may be used in the treatment of liver diseases, therapy and prevention of liver diseases in alcohol addics in order to maintain the function and structure of the liver as well as to regenerate the liver.

In the publication WO 2006/016143 it has been stated that a preparation consisting of alpha-ketoglutarates and a number of derivatives thereof, that was formerly used to activate HIFa hydroxylase in order to increase the alpha-ketoglutarate level, is now used to treat cancer and angiogenesis.

In accordance with publication WO 2006/062424 it is also possible to use 3-hydroxy-3 methylbutyrate in combination with alpha-ketoglutarate and a number of derivatives in the process of growth and mineralisation of skeleton in physiological conditions and in osteochondropathy processes in adult humans and in animals. The same product may be added to functional food and medicated food.

The publication WO 2006/016828 indicates the use of alpha-ketoglutarates and a number of derivatives as a pharmaceutical preparation and also as a food and animal feed additive improving functions of nerve cells and nervous system, minimising and preventing apoptosis of nerve cells, and protecting against nervous system diseases in adults and foetuses.

Pyridoxine alpha-ketoglutarate is known as an agent used in prophylaxis of acidosis in human medicine and veterinary medicine, in prophylaxis of all conditions leading to acidosis, as well as in pathologies where medicines decreasing lactic acid levels in blood are used.

Alpha-ketoglutarate is also used in medical practice in detoxificant in case of intoxication of an organism. The detoxifying action of alpha-ketoglutarate was used, for instance, in treating cyanide poisonings. Alpha-ketoglutarate as an anti-toxic agent prevents post-operational muscle catabolism and it is also used in hospitals in patients with parenteral feeding recommendation, where alpha-ketoglutarate is one of the compounds of the applied bolus. Alpha-ketoglutarate is also recommended for patients that suffer cerebrovascular stroke, and also with bum wounds, hypoxia, in those irradiated with X-rays or in the case of cataracts resulting from selenite poisoning.

Alpha-ketoglutarate combined with ornithine effectively protects the organism after small bowel transplantation against translocation of bacteria, as examined in mesenteric lymph nodes, the liver and spleen. See: de Oca J, Bettonica C, Cuadrado S, Vallet J, Martin E, Garcia A, Montanes T, Jaurrieta E. Effect of oral supplementation of ornithine-alpha-ketoglutarate on the intestinal barrier after orthotopic small bowel transplantation. Transplantation. 1997;63:636-639.

In rats in an experimentally induced post-trauma state, administration of ornithin-alpha-ketoglutarate reduces spreading of *E. coli* and destruction of tissue following LPS. It is assumed, that in humans after trauma, administration of this product may prevent sepsis and consequences thereof. See: Schlegel L, Coudray-Lucas C, Barbut F, Le Boucher J, Jardel A, Zarrabian S, Cynober L. Bacterial dissemination and metabolic changes in rats induced by endotoxemia following intestinal E. coli overgrowth are reduced by ornithine alpha-ketoglutarate administration. J Nutr. 2000;130:2897-2902.

Alfa-ketoglutarate is also used in animal breeding to improve amino acid absorption. It is administered to piglets to accelerate absorption of iron ions.

### Ureolytic bacteria

The range of nitrogen assimilating bacteria is wide: from non-pathogenic commensals, such as bacteria colonising skin, through non-pathogenic symbionts inhabiting mucous membrane of the gastrointestinal tract, to pathogenic bacteria, including *Helicobacter pylori* and bacteria causing urogenital system infections.

The most common infection caused by ureolytic bacteria is the *H. pylori* infection.

The common feature of ureolytic bacteria is their ability to use, by means of urease, urea present in their environment - mainly as a source of nitrogen necessary for survival live. Bacterial urease (urea aminohydrolase E.C.3.5.1.5) is a nickel-dependent multimer consisting of 2 or 3 subunits. The 3D crystallographic structure of some bacterial ureases has been discovered (*H pylori, Klebsiella aerogenes, Bacillus pasteurii*). A high degree of similarity of amino acid sequences indicates that all types of ureases originate from one parent protein, that they probably have a similar three-dimensional structure and that they maintain catalytic activity while hydrolysing urea to ammonia and carbon dioxide.

An example of nitrogen-assimilating bacteria by means of their own ureases are *Streptococcus salivarius* and *Actinomyces naeslundii* commonly present in the oral cavity and forming a biofilm.

The gastrointestinal tract has the biggest concentration of ureolytic bacteria. Microorganisms, including ureolytic ones, permanently colonise a surface of epithelium, and they are recognised as a natural intestinal microflora. This means, that access to urea is one of the critical factors of microorganism ecology in the gastrointestinal tract, i.e. it influences the quantity and quality of bacteria in this area. Through the maintenance of tissue integrity, the access to urea is one of the conditions of macroorganism's health. The same rule of homeostasis governs the occurrence of microorganisms colonising body surfaces.

Urea is also important as a substrate for pathogens' urease in the stabilisation phase of inflammation during *H. pylori* infection.

A common route of entry of pathogens into human and animal organisms is via the gastrointestinal tract with food, regardless of the place where infection develops further on. Infections with ureolytic microorganisms through the gastrointestinal tract show that urease plays a role in the pathogenesis of these infections. It was demonstrated that bacterial strains, for instance of the urease producing *Brucella* strains are resistant in the urea environment to the biocidal action of gastric juice with its strongly acidic conditions. Urease-negative mutants of these bacteria are on the other hand susceptible to such conditions, which results in a reduced number of bacteria after passage through the stomach. It may be concluded, that under such conditions urease protects brucellae against the effects of the gastric juice when they enter the organism orally. Having passed the barrier of stomach, bacteria are free to invade e.g. respiratory and urogenital systems and generate symptoms typical for brucellosis.

Ureolytic bacteria, even though they are not the main etiological factor in urinary system infections in healthy organisms, they are often related with infections in humans with urinary systems disorders. The consequence of urinary system infections with urease-producing microorganisms is staghorn calculus accompanied by supersaturation of urine with ammonium magnesium phosphate salts (struvite) and phosphate calcium salts, as well as by pathological processes within kidneys. Under physiological conditions urine does not contain these salts.

Another interesting mechanism in the pathogenesis of urogenital system infections is the maintenance of infections caused by urease-positive bacteria, such as *Ureaplasma ureolyticum* and other alkalophilic bacteria, e.g. *Bacillus pasteurii.* In the urea environment pathogens use ureolysis to generate their own ATP and thus they reproduce permanently. Even though *U. urealyticum* and other mycoplasmas are relatively rare, they may also cause dangerous and difficult to cure infections of the respiratory system in humans and animals, including fish.

It was also found, that urease-producing *Yersinia enterocolica* - a gastrointestinal tract pathogen may in some genetically handicaped people cause reactive arthritis and its reactivity is related to the chemical structure of the enzyme, its subunit UreB.

It should be noted that many bacteria capable of movement are ureolytic organisms responsible for formation of biofilm and mineralisation of deposits on catheters and other mechanical medical implements.

Urea metabolism is also believed to be related with infections in the mucous membranes of the oral cavity, including gingival diseases, formation of dental caries and tartar.

Formation of infectious stones is related with urinary system infections caused by bacteria of the following genii: *Proteus, Ureaplasma, Klebsiella, Pseudomonas, Staphylococcus, Providencia, Corynebacterium*. The most common cause of formation of infectious stones is the *P. mirabilis* bacteria. Another factor in formation of the stones are mycoplasmas usually reported to be related with genital tract, in particular its lower part (mainly vagina) infections. From the urethra in men the following organisms are isolated: *Mycoplasma hominis* and *U. urealyticum.* Colonisation of the urogenital system in both women and men by the bacteria leads to urinary system infections accompanied by formation of infectious stones in the bladder.

### Helicobacter pylori infections

*H. pylori* rods in humans are usually isolated from the stomach and duodenum. These Gram-negative, ureolytic, spiral-shaped bacteria formerly known as *Campylobacter pylori* are one of the etiological factors of gastritis and the formation of ulcers in stomach and duodenum. In 1983 Warren and Marschall, honoured in 2005 with a Nobel Prize, showed the cause-effect relationship between the occurrence of *H. pylori* bacteria in the gastrointestinal tract and chronic gastritis. See: Marshall BJ, Warren JR. Unidentified curved bacilli in the stomach of patients with gastritis and peptic ulceration. Lancet. 1984;1:1311-1315. Due to the fact that long-lasting infection evidently increases the risk of intestinal type adenocarcinoma, in the recent years *H. pylori* rods were declared by WHO as a carcinogenic factor (IARC. Schistosomes, liver flukes and *Helicobacter pylori*. IARC Working Group on the Evaluation of Carcinogenic Risks to Humans. Lyon, 7-14 June 1994. IARC Monogr Eval Carcinog Risks Hum. 1994;61:1-241.). Moreover, a relationship was found between *H. pylori* infection and pathologies in tissues and organs other than the stomach, for example in heart.

For a long time it was believed that stomach is free from bacteria because the permanent colonisation of mucous membrane in stomach is difficult due to the fact, that its pH is too low for growth of the majoritt of microorganisms. However, the urease produced by *H. pylori* allows this organism to form permanent colonies in stomach and intestine, while ureolysis is the main factor causing pathologies related with *H. pylori* infections. Urease deposited outside of the bacteria cell may constitute even up to 20 % of bacterial protein. The enzyme protects the bacteria against the acidic environment and prevents the lethal destruction of the bacterial cell envelope. Additionally, during the *H. pylori* infection ammonium ions released by urease during the course of urea decomposition have cytotoxic effects on stomach epithelium cells. In the presence of leukocytes and urea, bacterial urease generates monochloramine that may induce DNA mutagenesis, one of the factors involved in the development of cancer in the course of chronic *H. pylori* infection.

New species of ureolytic spiral bacteria are being isolated in humans and animals. However, so far it is not clear how other *Helicobacter* species occurring in humans should be connected with diseases of stomach (for instance *H. heilmannii*)*,* intestines (for instance *H. cinaed, H. canadensis*), liver (for instance *H. hepaticus, H. bilis*) or with systemic infections (for example *H. pullorum, Helicobacter* spp. *flexispira* taxon 8 *"F. rappini"*).

In recent years it has been found that the gastrointestinal tract (intestines, stomach, liver, and pancreas) in laboratory animals may be naturally colonised by bacteria of the *Helicobacter* genus: *H. bilis, H. ganmani, H. hepaticus, H. muridarum, H. mastomyrinus, H. rappini, H. rodentium, H. typhlonius.* Animals - both wild and domestic, permanently colonised with these microorganisms, do not manifest infection symptoms and no inflammatory processes are found in their internal organs in the course of autopsy. Microorganisms susceptible to environmental conditions continue to thrive due to permanent horizontal transmission of bacteria from one individual to another through faeces and saliva.

It has been found out that in humans *H. pylori* infection is present in 50% of the population and it seems to be related to the economic status and age of communities. It increases in adults, including almost entire populations in poorly developed countries. *H. pylori* is found in 90 - 100% of gastritis cases. Often chronic infection is found to be transformed into atrophic inflammation. In 10 % of those infected, serious pathologies develop. Infection with *H. pylori* is common both in children and in adults. Most often infection occurs in a childhood and the colonisation of the mucous membrane of the stomach with *H. pylori* rods is maintained throughout the live-time.

It is known that factors such as malnutrition, vitamin deficiency and smoking play a role in infection.

In 10 % of patients, applied therapy is ineffective due to existing and increasing resistance of bacteria to standard medicines. Some patients are also found to be re-infected with drug-resistant bacteria. The other ca. 10% of patients do not tolerate preparations from the group of proton pump inhibitors. In these patients adverse side effects are manifested.

Due to the systematically decreasing effectiveness of treatment and the difficulty thereof, regional gastrological associations in Europe, following the recommendation of the "Maastricht 2-2000" report, recommend appropriate diagnostic procedures to detect *H. pylori* and starting a treatment only where certain disease symptoms occur, such as: gastritis, stomach and duodenal ulcers, peptic ulcer confirmed in course of interviews, surgery due to peptic ulcer, pre-cancer changes (atrophic inflammation, metaplasia, dysplasia), stomach resection due to an early cancer, stomach cancer in family (up to 2^{nd} level of consanguinity), gastric hyperplastic adenomatous polyposis (after removal thereof), MALT lymphoma, long-term treatment with NSAIDs.

It was noticed that in humans *H. pylori* infection is accompanied by certain diseases, such as peptic ulcers, gastric lymphomas, chronic atrophic gastritis with intestinal metaplasia and stomach cancer.

In humans in the first stage of *H. pylori* infection an inflammation of the mucous membrane in the stomach and duodenum develops which should be fully eradicated with pharmacological measures. *H. pylori* infection treatment is still based on the introduction of substances which decrease secretion of gastric juice and raise the pH in the stomach. Such substances include proton pump inhibitors and certain antibiotics which eliminate the bacteria - clarythromicin, amoxicillin and chemical compounds - metronidazole (from the group of nitroimidazole derivatives).

In Europe the list of recommendations in the "Maastricht 2 - 2000" report published in 2002 does not include any *H. pylori* therapy without anti-bacterial measures such as antibiotics, and in terms of chemotherapeutic compounds - substances other than nitroimidazole derivatives. The therapy is complex, costly, sometimes badly tolerated and not always effective. In accordance to the recommendations of European regional Gastroenterological Associations a treatment may be based on antibiotics and chemotherapeutic compounds (clarythromicin (2 x 500) and amoxicillin (2 x 1 g) or metronidazole (2 x 500 mg), as well as proton pump inhibitors.

For example:
First-time therapy. A seven-day treatment cycle:
   1. Medicine reducing the secretion of gastric juice; a double dose of a compound belonging to the group of proton pump inhibitors (PPI) - for instance omeprazol 2 x day, 20 mg
   2. Antibiotic I - for instance amoxicillin, 2 x day 1 g
   3. Antibiotic II - for instance clarythromicin, 2 x day 0.5 g
Second-time therapy.
   1. Medicine reducing the secretion of gastric juice; a double dose of a compound belonging to the group of proton pump inhibitors (PPI) - for instance lanzoprazol 2 x day, 30 mg
   2. Antibiotic I - for instance maintain amoxicillin, 2 x day 1 g
   3. Antibiotic II - other antibiotic or chemical compound - for instance metronidazol, 2 x day 0.5 g
   4. bismuth compounds (citrate).

Due to the epidemic spread of *H. pylori* in different parts of the world, there is a great and permanent demand for preparations reducing pathologies related to the infection ; there is great pressure upon the medical community to identify such preparations.

The main object of the invention is to provide a new preparation for treatment and prophylaxis of diseases caused by urolytic bacteria, to be used in particular to regulate ureolytic flora of intestines, to regulate ureolytic flora of oral cavity, to inhibit passage of pathogenic ureolytic bacteria through a digesting stomach, to prevent formation of deposits and infectious stones in the urinary system - in humans and animals, in particular in dogs and cats, and inother domestic animals.

The object of the invention is to provide a new agent for control of undesired growth of ureolytic bacteria in humans and animals.

A specific aim of the invention is to provide a new preparation for treatment and prophylaxis of diseases caused by *H. pylori.*

Still further object of the invention is to provide a new preparation for inhibiting growth of ureolytic bacteria, in particular of *Ureaplasma* and other mycoplasmas causing fish diseases, for use in particular as a prophylaxis of gill inflammation caused by ureolytic bacteria in carp and carp fry and other fresh water and sea fish.

Besides, it is the object of the invention is to provide a new preparation for preventing formation of biofilm and mineralisation of deposits on catheters and other medical equipment.

It is also the aim of the invention is to provide a new preparation for reduction of tartar formation and inhibition of development of caries.

Furthermore, it is a further aim of the invention is to ensure new dietary supplements, special medicated food product and food/feed additives preventing and/or inhibiting colonisation of vertebrates, including humans, mammals, birds, amphibian and fish, by undesired ureolytic bacteria, in particular colonisation of humans and of domestic animals by *Helicobacter pylori.*

These aims were achieved by providing a solution according to the invention as presented in the attached patent claims.

The achievement of the above mentioned aims is assured in accordance with the invention by use of alpha-ketoglutarate as an active substance in therapeutically or prophylactically effective doses to produce a therapeutic or prophylactic medical preparation or a dietary supplement, special medicated food product and food/feed additive or else a personal hygiene products for everyday use.

Therapeutic and/or prophylactically effective dosages range from 0.001g to 0.2 g/kg of body mass/day when administered intragastrically or orally.

Until now, salts of alpha-ketoglutaric acid have never been used in humans or in animals to treat bacterial infections, including *H. pylori* infections.

The advantage of this invention is the availability of alpha-ketoglutarate, its well examined activities within the organisms, as well as the fact that this substance is approved for use in other medical and prophylactic applications.

The invention is further explained in the following detail description and with reference to the enclosed drawings.

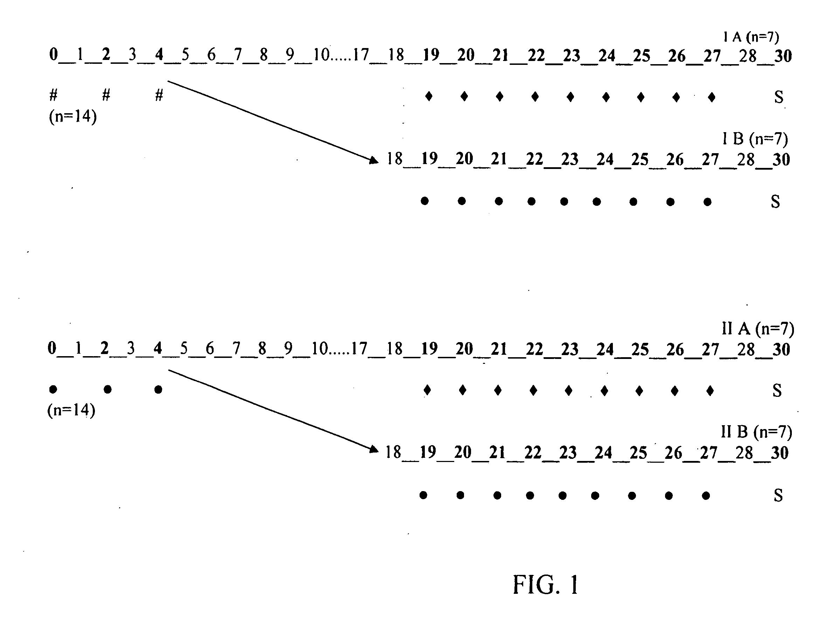

In the drawings enclosed, Fig.1 shows the scheme of the experimental mice infectious model with *H. pylori* bacteria, used to study the relationship between colonisation level of mouse stomach mucosa (n=28) by *H. pylori* and the mice treated intragastrically with salts of alpha-ketoglutaric acid.

Fig. 2 shows the scheme of the experimental mice infectious model with *H. pylori* bacteria, used to study the relationship between colonisation level of mouse stomach mucosa (n=48) by *H. pylori* and the mice treated intragastrically with salts of alpha-ketoglutaric acid.

Fig.3 shows mobility of PCR products related to 16S rDNA *Helicobacter* genus bacteria in electrical field assayed with DGGE technique.

The terms used throughout the description and the attached patent claims have the following meaning:

The term "alpha-ketoglutarate" as used herein (sometimes referred to as AKG), refers to the compound releasing an active anion from the acid known as 2-oksopentanedioic, 2-oksoglutaric, alpha-oksoglutaric, alpha-oksopentanedioic, 2-ketoglutaric, 2-okso-1,5-pentanedioic, 2-oksopentanedioic, or 2-okso-glutaric. The examples of such compounds are salts, additive salts, esters, amides, imides of alpha-ketoglutaric acid and prodrugs thereof. Alpha-ketoglutarate exerts an inhibitory action on colonisation and preserves mucosa colonisation by ureolytic bacteria in vertebrates, including humans, mammals, birds, amphibian and fish.

The term "medical preparation" used here refers to a composition comprising a therapeutically effective quantity of alpha-ketoglutarate to be used in new medical or prophylactic indications covered by this invention.

The term "therapeutically effective" denotes such a specific quantity of a derivative, in particular, a salt of alpha-ketogluteric acid, that under *in vivo* conditions presented in this description has a therapeutic effect, i.e. reduces and inhibits colonisation of the mucous membrane by ureolytic bacteria in vertebrates, including humans, mammals, birds, amphibian and fish. Therapeutic or prophylactic effectiveness is achieved by introducing the above mentioned medical preparation in a solid or liquid form, with or without a vehicle, diluent, additive, or else as a component of a pharmaceutical composition, into organisms of vertebrates, including humans, mammals, birds, amphibian and fish. The preparation is administered in quantities sufficient to reduce and prevent ureolytic bacteria infections. Alternatively, therapeutically effective quantity of the medical preparation heals the infections caused by ureolytic bacteria.

Depending on the desired effects, the quantity of the preparation may differ, according to its specific action on the ureolytic bacteria in the target place. A dose of the preparation may comprise a defined quantity of a substance calculated in such a way as to bring about the expected therapeutic results. Dosage is given in terms of a pure active substance taking into account its chemical structure and presence of additional substances, such as vehicle, diluent, adjuvants and other permitted pharmaceutically acceptable additives. Desired therapeutic effect and thus the recommended dose may be determined by means of known methods by an employee of medical or veterinary service, mainly based on such parameters as age, weight, sex of the patient, other accompanying infections and diseases, in accordance with good medical practice.

The term "administration of the medical preparation" refers to prophylactic or therapeutic response to the abovementioned diseases with an appropriate route of administration adjusted to the place, type and intensity of infection, taking into the account the route of entry of harmful ureolytic bacteria into the organism.

The term "inhibiting of colonisation" relates to the reduction of spread and/or severity of infection within mucous membranes or other tissues, caused by ureolytic bacteria, or complete eradication of the infecting factor - leading to the reduction and/or prevention of further development of infection in organisms of vertebrates, including humans, mammals, birds, amphibian and fish.

The term "preventing of colonisation" refers to the prevention of the development of harmful ureolytic bacteria, when the bacteria get into contact with the mucous membrane of a vertebrate. In the case of an effective prevention of colonisation, infection does not occur or mucous membranes of the vertebrates - including humans, mammals, birds, amphibian and fish - are infected with considerable delay in comparison to a scenario without colonisation prevention.

The term "dietary supplement" means a concentrated source of nutrients or other substances with nutritious or physiological effect, the use of which contributes to supplementing everyday diet deficient in certain beneficial components. Dietary supplements are produced in an easy-to-use form, e.g. tablets, capsules or liquids in one-dose packages.

The term "medicated food product" means a food product with form and recipe typical for the product, containing an additional substance of specific therapeutic or prophylactic value.

The term "food/feed additive" relates to a product containing an active substance, pure or in a composition, in solid or liquid form, with or without vehicles, buffers, detergents, solubilizers, antioxidants, preservatives and other additives substances corresponding with the active substance profile, and approved for usage in food.

### Detailed description of the invention

The invention relates to the use of alpha-ketoglutarate in the form of a single compound or a mixture of various compounds, for manufacturing of medical preparation for use in prophylaxis and/or treatment of diseases caused by pathogenic ureolytic bacteria in vertebrates, including humans, mammals, birds, amphibian and fish.

Prophylactic or therapeutic effect of a administration of the preparation manufactured in accordance with the invention is reached, when from 0.001 to 0.2 g of alpha-ketoglutarate is used per 1 kilogram of body mass/day.

This invention is based on the observation that *Helicobacter pylori* bacteria are able to survive in the highly acidic environment of the higher organisms' stomachs.

A generally known property of *H. pylori* is its ability to survive in low pH environment due to the activity of urease which hydrolyses urea present in the mucous membrane of stomach and in the gastric juice (Saidijam M, Psakis G, Clough JL, Meuller J, Suzuki S, Hoyle CJ, Palmer SL, Morrison SM, Pos MK, Essenberg RC, Maiden MC, Abu-bakr A, Baumberg SG, Neyfakh AA, Griffith JK, Sachs G, Scott D, Weeks D, Melchers K. Gastric habitation by Helicobacter pylori: insights into acid adaptation. Trends Pharmacol Sci. 2000;21:413-416, Sachs G, Weeks DL, Melchers K, Scott DR. The gastric biology of Helicobacter pylori. Annu Rev Physiol. 2003;65:349-369, Sidebotham RL, Worku ML, Karim QN, Dhir NK, Baron JH. How Helicobacter pylori urease may affect external pH and influence growth and motility in the mucus environment: evidence from in-vitro studies. Eur J Gastroenterol Hepatol. 2003;15:395-401.). The scheme of individual reactions is as follows:
(1) H₂NCONH₂ + H₂O → CO₂ + 2 NH₃
(2) CO₂ +H₂O → H₂CO₃
(3) H₂CO₃ + 2NH₃ → NH₄⁺ + HCO₃⁻ + NH₃
(4) H⁺Cl⁻ +NH₃ → NH₄⁺ + Cl⁻

In result of the urea decomposition process is formation of ammonia reacting immediately with hydrochloric acid so that in the tissue (mucous membrane of the stomach) micro-environment pH is locally increased. As it is known, under natural conditions, the mucous membrane of the stomach is acidic, due to the HCl production in parietal cells.

Even though *H. pylori* rods are sensitive organisms difficult to be grown *in vitro,* the low pH in the stomach - lethal to other bacteria - may paradoxically support *H. pylori* colonisation. This is mainly due to the presence of endogenous urea decomposed by bacterial urease (an enzyme produced by *H. pylori*) to ammonia which increases pH in the micro-environment of the bacteria. Thus, the lethal influence of the acidic environment on *H. pylori* is eliminated. It is even believed (Nakazawa T. Growth cycle of Helicobacter pylori in gastric mucous layer. Keio J Med. 2002;51,S2:15-19.) that the *H. pylori* growth cycle in the gastric mucous layer is stimulated by ureolytic properties of the microorganisms. It was proved, that on the cell level urease mRNA is stabilised and destabilised depending on pH of the environment. It was assumed that the bacteria use nutrients from degraded cells in order to grow and colonise a region where pH has already been changed. Urease activation is accompanied by the opening of a pH-dependent UreI channel in bacterial cell which facilitates urea hydrolysis (Weeks DL, Eskandari S, Scott DR, Sachs G. A H+-gated urea channel: the link between Helicobacter pylori urease and gastric colonization. Science. 2000;287:482-485, Weeks DL, Sachs G. Sites of pH regulation of the urea channel of Helicobacter pylori. Mol Microbiol. 2001;40:1249-1259.). It was also noticed that *H. pylori* has the ability to move towards greater concentrations of urea, and this chemotaxis accelerates the urea hydrolysis process. This explains the second round of the growth cycle and in consequence the stabilisation of the infection in the stomach (Scott DR, Marcus EA, Weeks DL, Sachs G. Mechanisms of acid resistance due to the urease system of Helicobacter pylori. Gastroenterology. 2002;123:187-195, Scott DR, Marcus EA, Weeks DL, Lee A, Melchers K, Sachs G. Expression of the Helicobacter pylori ureI gene is required for acidic pH activation of cytoplasmic urease. Infect Immun. 2000;68:470-477, Voland P, Weeks DL, Marcus EA, Prinz C, Sachs G, Scott D. Interactions among the seven Helicobacter pylori proteins encoded by the urease gene cluster. Am J Physiol Gastrointest Liver Physiol. 2003;284:96-106.).

On the other hand, it is known that during the course of amino acid degradation - as a result of oxidizing deamination the α-amine groups are transferred to α-keto acids and it is accompanied by the detachment of ammonium ion. Ammonium ions produced as a result of amino acid decomposition partly participate in the biosynthesis of nitrogen compounds, and the remaining ones are removed outside of the organism after transformation into urea. In urea one of the nitrogen atoms originates directly from the ammonium ion.

Under natural conditions urea may diffuse freely from the place where it is produced, i.e. from hepatocytes, to the entire digestive system, this is due to the low molecular mass of this compound (Mᵣ 60). Due to the strong toxicity of the ammonium ion the organism protects itself by employing the ion in synthesis of low-toxicity compounds, such as urea (ureotelic organisms - mammals). Ureotelic organisms are not able to store nitrogen: which relates to proteins, amino acids and ammonia.

In an organism, free ammonia occurs in insignificant quantities in spite of continuous deamination of amino acids. Some ammonia is immediately bound by glutamate and asparaginate. Some ammonia is excreted through kidneys in the form of ammonium ions. The majority of the toxic ammonia is converted into urea in the liver in the ornithine cycle.

At present it was unexpectedly found that after administering alpha-ketoglutarate to the stomach, the population of *Helicobacter pylori* decreases. Under conditions of an *in vivo* experiment the level of ammonium ions is relatively low and removal thereof by alpha-ketoglutarate (immediate binding into glutamate and asparaginate), in spite of improved anion transport to gastric mucous layer cells (through DC transporter), leads to the death of ureolytic bacteria. Production of ammonium ions from urea, necessary for survival of *H. pylori* in the stomach, does not cover the needs of the rods, because the ammonium ion is immediately utilised, with involvement of alpha-ketoglutarate, in the synthesis of glutamate and other amino acid compounds.

It is still unclear why *H. pylori* produces excessive in relation to the substrate quantities of urease.. Bacterial urease is a nickel-dependent enzyme with nickel divalent cation posttranslationally incorporated. It is assumed, that in order to maintain an appropriate quantity of active enzyme *H. pylori* accumulates it to safeguard ureolytic activity of descendant cells growing even under conditions of nickel deficiency. Due to intensive up-take of alpha-ketoglutaric acid salts in the stomach, *H. pylori* may also compete for access to these salts which causes an intensive production of bacterial urease in the first stage of infection.

Due to the epidemic spreading of *H. pylori* infections, searching for preparations limiting pathologies resulting from the infection is a subject matter of numerous reports. See: El-Omar EM. Mechanisms of increased acid secretion after eradication of Helicobacter pylori infection. Gut. 2006;55:144-146., Wotherspoon AC, Ortiz-Hidalgo C, Falzon MR, Isaacson PG. Wotherspoon AC, Ortiz-Hidalgo C, Falzon MR, Isaacson PG. Helicobacter pylori-associated gastritis and primary B-cell gastric lymphoma. Lancet. 1991;338:1175-1176.

Alpha-ketoglutarate participates, alongside ammonium ions, in the synthesis of certain amino acids, such as glutamic acid, and next glutamine, in the following reaction:

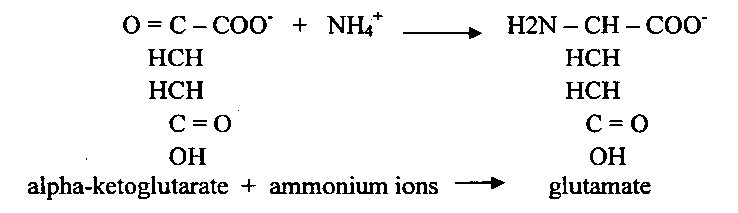

It was this ability of alpha-ketoglutarate to bind ammonium ions that instigated the research on the new medical application of this well-known substance.

It was assumed that the above mentioned reaction is a pathway of binding ammonium ions competitive to synthesis of urea being a necessary source of nitrogen for *H. pylori* in the gastric environment or for other ureolytic bacteria, for instance in the urogenital system.

Due to the lack of reports concerning harmful actions of alpha-ketoglutarate on the mucous membrane of the stomach and small intestine in healthy volunteers, a series of experiments were conducted on healthy laboratory animals to establish the influence of alpha-ketoglutarate on *H. pylori* colonisation of stomach and small intestine in healthy laboratory animals.

Unexpectedly, the results confirmed the above mentioned ssumptions showed the following:
1. There were no morphological changes in the thickening of the mucosa in the stomach of mice infected with *H. pylori* and following inoculation with alpha-ketoglutarate, or in the thickening of the mucosa in the small intestine and in the villus width and crypt depth in animals tested.
2. There were no changes in the amount of lactic acid bacteria isolated from scraped stomach mucosa (antral part) in animals tested, including those samples from mice infected with *H. pylori* and following inoculation with alpha-ketoglutarate.
3. A 17 % (p < 0.01) reduction in the number of gastrin - gastric hormone - producing cells localised in the pyloric region of the stomach glands was observed in animals infected with *H. pylori* and following inoculation with alpha-ketoglutarate in contrast to the control group (challenged with phosphate buffer - PBS). In animals infected with *H. pylori* and following inoculation with alpha-ketoglutarate a decreasing level of blood gastrin occurred (from 21.8 pM - 24.7 pM up to 12.8 pM - 15.6 pM) (p < 0.05), most probably as a result of the reduced number of gastrin-producing cells in stomach mucosa. This fact indicates an indirect (through histamin) inhibitory interaction between alpha-ketoglutarate and proton pomp activity. Its hyperactivity is manifesting in reflux diseases.
4. Some tendency towards a decreasing number of cholecystokinin (CCK) tissue hormone producing cells was noticed in the small intestine of animals treated with alpha-ketoglutarate. There is a slope in 30 % (without statistical differences, p = 0.1) as compared to this same intestine segment of mice not infected, inoculated only with PBS. There were no significant differences in the number of CCK-producing cells in the small intestine of mice infected with *H. pylori,* following the challenge with alpha-ketoglutarate as compared to the mice inoculated only with alpha-ketoglutarate (p = 0.25). A low CCK blood level (from 3.1 pM - 4.0 pM up to 1.9 pM - 2.5 pM) (p < 0.05) in animals infected with *H. pylori* and inoculated with *H. pylori,* and following with alpha-ketoglutarate can be connected to the decreasing number of CCK producing cells in the small intestine. Therefore the low CCK level can stimulate stomach emptying in increasing frequency; it is stated by medical service as a factor avoiding further colonisation by *H. pylori* and infection development.

Bactericidal effect of alpha-ketoglutarate on *H. pylori* rods.

It was presently unexpectedly found that the salt of alpha-ketoglutaric acid inhibits the process of *H. pylori* colonisation of the gastrointestinal tract of mammals. The present studies show that the average number of colonies isolated from mucosa in the pyloric part of the stomach in mice infected only with *H. pylori* - on the thirtieth day after administering the first dose of suspension of the bacteria cells was equal to 7.8 x 10² ± 5.0 x 10¹. In the group of laboratory animals which after 14 days from infection received an intragastric dose of the salt of alpha-ketoglutaric acid for 9 consecutive days, the average number of isolated colonies was only 3.8 x 10² ± 5.0 x 10¹. Nine intragastric administrations of alpha-ketogluterate for nine consecutive days, which started 14 days after the last infective dose with *H. pylori* resulted in a 49 % decrease in the gastric mucous layer colonisation by the bacteria. The results prove that the salts of alpha-ketoglutaric acid inhibit colonisation of stomach by *H. pylori.*

In another experiment it was found that the average number of colonies isolated from the mucosa in the pyloric part of the stomach of mice infected solely with *H. pylori* on the twentieth day after the first administration of suspension of bacterial cells, was equal to 4.3 x 10² ± 5.0 x 10¹. In the group of laboratory animals which received alpha-ketoglutarate intragastrically for 3 consecutive days, 8 days after infection, no *H. pylori* colonies were found. Three intragastric administrations of alpha-ketoglutarate continued for 3 consecutive days, which started 8 days after the last infective dose of *H. pylori,* completely stopped colonisation and fully eradicated *H. pylori* in mice gastric mucosa.

In course of the observation, the gastric microflora altered and in mice infected with *H. pylori* DNA of *H. bilis* was also found; in mice additionally inoculated with alpha-ketoglutarate - only DNA of *H. rodentium, H. bilis, H. hepaticus* were found; in the control group of mice, to whom salts of alpha-ketoglutaric acid were administered, DNA of *H. hepaticus* i *H. rodentium* was identified. In mice treated with PBS DNA of *Helicobacter* bacteria was not found.

The results were obtained by means of routine diagnostic methods supported with classic techniques which assume that a living microorganism must be cultured in order to confirm the presence of bacteria in a tissue (fulfilment of Koch's postulates). Additionally the studies were supported with DNA detection methods for *H. pylori* and other non-pathogenic bacteria of the *Helicobacter* genus. PCR followed by electrophoretical separation of PCR products by means of Denaturing Gradient Gel Electrophoresis (DGGE), and sequencing of products intended to examine the DNA composition is a method for detecting changes that were described here. In practice, in course of sequencing no *H. pylori* DNA was found (sensitive level: ethidium bromide staining) in mice treated with alpha-ketoglutarate.

Alpha-ketoglutarate is thus an ideal active substance for a large and varied group of patients that require prophylaxis and treatment against *H. pylori,* and urogenital system infections caused by ureolytic bacteria.

Mechanism of bactericidal action of alpha-ketoglutarate against ureolytic bacteria - quite different from those mechanisms known so far, will allow safe eradication of these microorganisms without fear of induction and increase of drug resistance in ureolytic bacteria. In practice, it will result in reduction of reinfections, superinfections and difficulties in treatment with antibiotics.

As it has been established so far, alpha-ketoglutarate supports, or is an alternative to, standard antibiotic treatment. Also, in cases of cachexia it may serve to balance the natural ureolytic microflora.

In accordance with the invention, alpha-ketoglutarate and/or appropriate precursors releasing under *in vivo* conditions anions of alpha-ketoglutaric acid will be used to manufacture a medical preparation to be used in prophylaxis and/or treatment of diseases caused by ureolytic bacteria.

The preparation obtained in accordance with the invention is useful in particular to prevent and/or inhibit *Helicobacter pylori*_colonisation.

The following salts are preferably used as alpha-ketoglutarate mono- and disubstituted salts of alpha-ketoglutaric acid and alkali metals and/or alkaline earth metals, and/or chitosan. The preferred salts are sodium salt and/or calcium salt.

Alpha-ketoglutarate, in accordance with the invention, may be used in humans and animals as a medical preparation, dietary supplement, special medicated food product and/or food/feed additive, depending on conditions, for inhibition of *H. pylori* colonisation in humans and animals, in order to prevent *H. pylori* infection and consequences thereof, or in order to alleviate *H. pylori* infection and consequences thereof.

Alpha-ketoglutarate may be administered together with known vehicles and additives that are approved for pharmaceutical use and are compatible with the selected alpha-ketoglutarate precursors. Appropriate additives include, for instance, water, saline, dextrose, glycerol, ethanol or other similar substances or combinations thereof. Moreover, where desired, the preparation may contain additional substances such as, for instance, wetting agents, emulsifiers, pH modulators, buffers and other.

In accordance with the invention, the preparation containing alpha-ketoglutarate may be solid and/or liquid, depending on the intended route of administration.

The invention relates also to the use of alpha-ketoglutarate for manufacturing preparations for treatment and prophylaxis of other ureolytic bacteria infections. Examples of further medical applications of alpha-ketoglutarate include the use of alpha-ketoglutarate to manufacture a preparation that inhibits passage of pathogenic ureolytic bacteria through the stomach, a preparation preventing formation of deposits and infectious stones in the urinary system, a preparation reducing formation of biofilm and mineralisation of deposits on catheters and other medical equipment, and also a preparation inhibiting growth of other pathogenic ureolytic bacteria in the urogenital system, to be used in the form of urethral infusions, tablets, irrigation liquids, intravaginal tablets.

In accordance with the present invention, alpha-ketoglutarate may also be used in dogs, cats and domesticated animals, in the form of bladder infusions in bacterial infections caused by ureolytic bacteria.

A further example of a new use of alpha-ketoglutarate is a use for manufacturing of a preparation regulating ureolytic flora of the oral cavity, reducing formation of tartar and inhibiting development of dental caries. This product may have the form of a chewing gum or a tooth paste.

Alpha-ketoglutarate finds also its further new use according to the invention, for manufacturing a preparation inhibiting growth of ureolytic bacteria, in particular *Ureaplasma* and other mycoplasmas causing infections in fish. In this respect, the new use of alpha-ketoglutarate is use for manufacturing a preparation for preventing inflammation of gills caused by the above mentioned ureolytic bacteria in carp and carp fry, and in other fresh water and sea fish.

The invention also covers the use of alpha-ketoglutarate in production of dietary supplements, special medicated food products, and food/feed additives used to prevent and/or inhibit *Helicobacter pylori* colonisation.

Examples below provide a better explanation of this invention.

### Example 1.

Laboratory experimental animals twenty eight 6 week-old BALB/cA (female) mice weighing 25 ± 2 g (fig. 1). Fourteen mice were challenged through a tube (outer diameter 1.3 mm) 3 times with one day intervals with 0.2 ml suspension of *H. pylori* cells, strain 119/95 at the concentration 10⁹ cfu/ml. Two weeks after the last challenge, seven mice were inoculated intragastrically for nine successive days by the same method with a solution of calcium or sodium salt of alpha-ketoglutaric acid (0.2 ml, at concentration 30 mM) (group I A). The remaining seven mice were sham-treated with 0.01 M phosphorate buffer - PBS (group I B) as animals from group I A.

Further fourteen mice from group II A and II B were treated with 0.2 ml PBS according to the scheme operating by the infection of the animals with *H. pylori.* Two weeks after the above treatment seven mice were continuously inoculated with PBS (0.2 ml) for three consecutive days. The remaining seven mice were treated with a solution of salts of alpha-ketoglutaric acid (0.2 ml, concentration 30 mM) (group II A).

On the 30^{th} day of the experiment all mice were sacrificed using CO₂. For further analyses blood and stomach samples from mice infected with *H. pylori* with or without the following inoculation with salts of alpha-ketoglutaric acid were collected. Scheme of the experimental mice infectious model with *H. pylori* bacteria has been presented in fig. 1. In this experiment the relationship between colonisation level of mouse stomach mucosa (n=28) by *H. pylori* and the mice treated intragastrically with salts of alpha-ketoglutaric acid were studied in accordance with the time regime as described above..

In fig.1 the following abbreviations were used. Mice from experimental groups (n=28) were inoculated intragastrically with the following preparations: suspension of *H*. *pylori* cells: #; solution of salts of alpha-ketoglutaric acid: ◆; solution of PBS: •. The name of the strain, concentration and volume of inoculum (*H. pylori*) and concentrations and doses of: salts of alpha-ketoglutaric acid and PBS, correspond to the above information. Bolded figuresaccompanied by the S letter indicate autopsy days. Autopsies were carried out in accordance with commonly binding standards.

Blood samples from all animals tested were smeared on GAB-CAMP agar plates and incubated at temp. 37°C, under microaerophilic conditions for 7 - 10 days. From the half of the antrum gastric mucosa was scraped off and mixed with 500 µl PBS sterilized. After homogenate balancing it was noticed that the amount of the scraped mucosa from the half of the antrum was generally between 40 µg a 50 µg. For calculation of number of *H. pylori* cells, a 100 µl of gastric mucosa homogenate was smeared onto GAB-CAMP agar plates and incubated at temp. 37°C, for 5 - 10 days, under microaerophilic conditions. Homogenized mucosa samples from each mouse were examined in triplicate. The results are presented as the mean value ± SD. The presence of *H. pylori* was identified by colony morphology and urease-, oxidase- and catalase tests, as well as by morphological examination of cells by Gram-staining.

There were no isolated *H. pylori* from tissue of animals inoculated intragastrically with PBS and with salts of alpha-ketoglutaric acid (Group II A in fig. 1), or with PBS (Group II B in fig. 1).

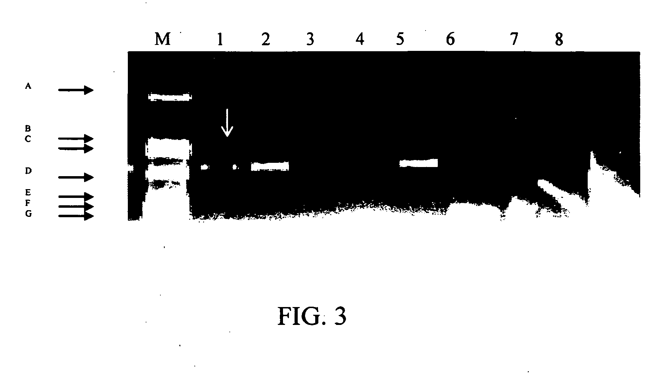

From stomach samples of animals inoculated with *H. pylori* and then following with salts of alpha-ketoglutaric acid (Group I A in fig. 1) or PBS (Group I B in fig. 1) bacteria were isolated between the 5^{th} and 10^{th} day of incubation. The number of colonies (mean ± SD) isolated from the antral part of the gastric mucosa from the mice exclusively infected with *H. pylori* was 7.8 x 10² ± 5.0 x 10¹ whereas after intragastric treatment of mice with salts of alpha-ketoglutaric acid, according to the protocol presented - the number of colonies (mean ± SD) isolated was 3.8 x 10² ± 5.0 x 10¹ (fig. 3). Nine times, during 9 consecutive days, intragastric induction of salts of alpha-ketoglutaric acid, which has been started after 14 days interval from the last infective dose of *H. pylori* bacteria for mouse, caused the reduction by 49 % of a degree of gastric mucosa colonisation by *H*. *pylori.* It is interpreted as inhibitory action of salts of alpha-ketoglutaric acid on colonisation stage of stomach by *H. pylori.*

From the blood samples taken from the mice neither *H. pylori* nor any other bacteria were isolated.

Results obtained are presented below in Table 1.

**Table 1. H. pylori bacteria isolated from homogenates of stomach mucosa of mice non-infected and infected with H. pylori with or without the following inoculation with the salts of alpha-ketoglutaric acid**

| Inoculates * | *H. pylori* cultivated ** | |
|---|---|---|
| | Number of mice inoculated in group | Number of bacteria (cfu)/mouse |
| *H. pylori* + PBS (Group I B) | 7/7 | 7.8 x 10² ± 5.0 x 10¹ |
| PBS (Group II B) | 0/7 | 0 |
| *H. pylori* + AKG (Group I A) | 7/7 | 3.8 x 10² ± 5.0 x 10¹ |
| PBS + AKG (Group II A) | 0/7 | 0 |

| | | |
|---|---|---|
| */ according to the scheme of fig. 1. **/ bacteria were cultivated on GAB-CAMP solid plates. Animals exclusively infected with *H. pylori* (*H. pylori* + PBS), infected with *H*. *pylori* with following inoculation of salts of alpha-ketoglutaric acid (*H. pylori* + AKG) and control groups non infected animals, inoculated only with PBS or with PBS and salts of alpha-ketoglutaric acid (PBS + AKG). | | |

### Example 2.

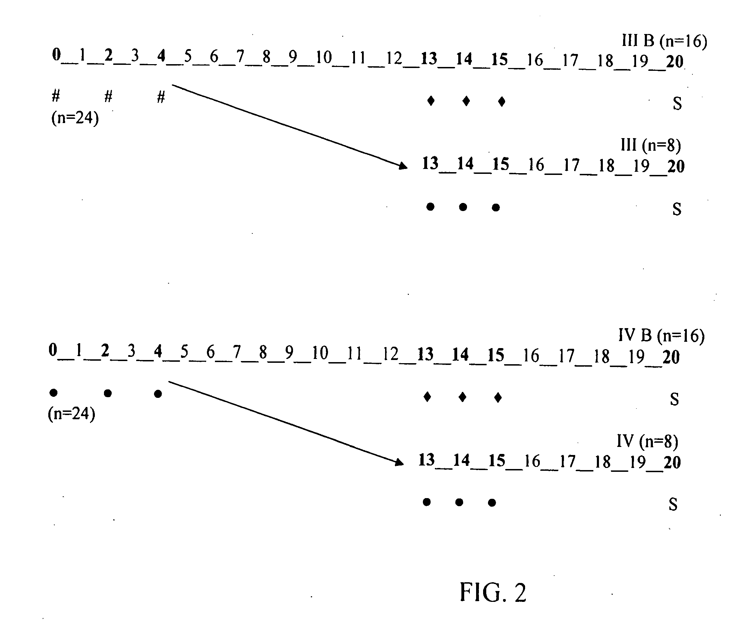

Laboratory experimental animals forty eight, 6 weeks old BALB/cA (female) mice weighing 25 ± 2 g (fig. 2). Twenty four mice (fig. 2) from group III B and III were challenged intragastrically 3 times with one day intervals with 0.2 ml suspension of *H*. *pylori* cells, strain 119/95 at the concentration 10⁹ cfu/ml. Eight days after the last challenge, sixteen mice were inoculated intragastrically through the tube for three successive days with 0.2 ml of 30 mM solution of salts of alpha-ketoglutaric acid (group III B). The remaining eight mice were sham-treated with 0.2 ml of 0.01 M PBS (group III) according to the procedure with group III.

The remaining twenty four mice (fig. 2) were inoculated intragastrically 3 times with one day intervals with 0.2 ml 0.01 M PBS. Eight days after above treatment sixteen mice were inoculated with the tube for three consecutive days with salts of alpha-glutaric acid (0.2 ml, concentration 30 mM) (group IV B). The remaining eight mice were treated with 0.2 ml 0.01 M PBS (group IV).

On the 20^{th} day of the experiment all mice were sacrificed using CO₂. Stomach and blood were samples were collected for the further studies.

In fig. 2 the following abbreviations were used. Mice from experimental groups (n=76) were inoculated intragastrically with following preparations: suspension of *H. pylori* cells: #; solution of salts of alpha-ketoglutaric acid: ◆; solution of PBS: •. The name of the strain, concentration and volume of inoculum (*H. pylori*) and concentrations and doses of: salts of alpha-ketoglutaric acid and PBS corresponding to the above text. Bold letters determined as the S letter indicate autopsy days. Autopsies were carried out in accordance with common principles.

From samples collected, as it is demonstrated in example 1, *H. pylori* bacteria were cultivated on GAB-CAMP agar plates. DNA was also isolated to perform PCR with primers (5'-CTATGACGGGTATCCGGC-3', N16S1R: 5'-CTCACGACACGAGCTGAC-3') recognizing 16S rDNA fragment in DNA of bacteria from *Helicobacter* genus. Afterwards PCR products in size 470 bp were separated according to denaturating gradient gel electrophoresis technique (DGGE) and sequenced. DGGE analysis was performed in 9%, polyacrylamide gel (acrylamide/bisacrylamide solution in rate 37.5:1). Electrophoresis run at a temp of 60°C in 125 V during 16 h.

Results obtained are presented below in Table 2.

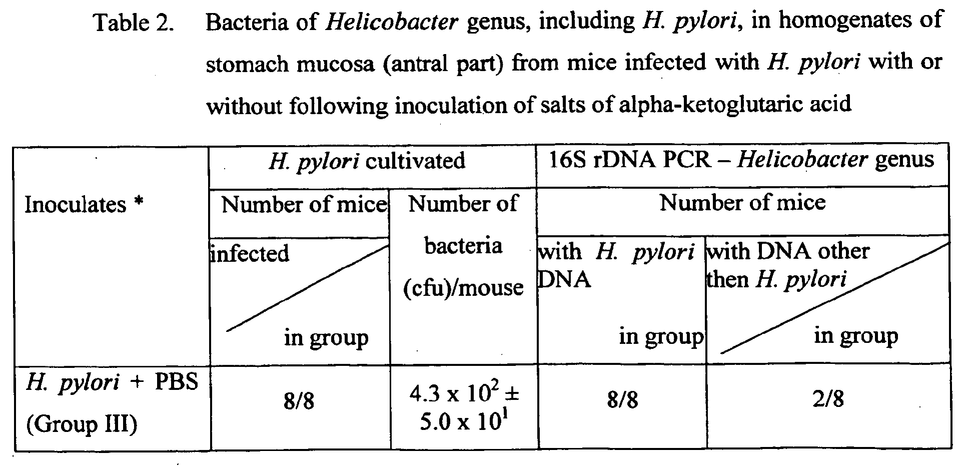

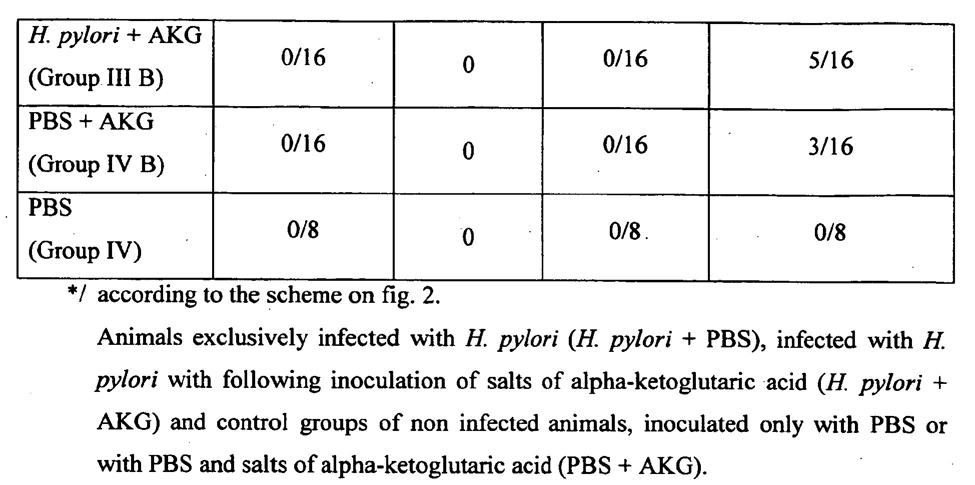

*H. pylori* was isolated from 8 stomach samples of 8 mice infected with *H. pylori* + PBS (Group III in fig. 2) (Table 2). In DNA isolated from the same stomach samples 16S rDNA *H. pylori* specific fragment was identified employing PCR.

From stomach samples obtained from 16 mice infected with *H. pylori* and treated with salts of alpha-ketoglutaric acid (Group III B in fig. 2) either *H. pylori* culture or sequences specific for *H. pylori* in PCR products was not found (Table 2).

No *H. pylori* rods were cultured from blood samples taken from these animals. With selected primers, DNA isolated from blood and from stomach of 8 mice challenged with PBS (Group IV in fig. 2) did not amplify.

In the enclosed figure Fig. 3 mobility of PCR products typical for 16S rDNA fragment of bacteria from *Helicobacter* genus in a field of electrical current evaluated with DGGE technigue was illustrated. A: *H. muridorum,* B: *H. bilis,* C: *H. pullorum,* D: *H. pylori,* E: *Helicobacter* spp. *flexispira* taxon 8 *"F. rappini",* F: *H. hepaticus,* G: *H. bizzozeronii* served as markers. Arrow indicates DNA of *H. bilis.* Letters from 1 to 8 estimate paths migrating PCR products from present example 2.

In 16 PCR products extracted from the antral part of the stomach of mice infected with *H. pylori* and afterwards treated or not treated with salts of alpha-ketoglutaric acid or from mice not infected, 19 DNA fragments of 470 bp in size were detected. Results are shown in Fig.3 and in Table 3 below. Sequencing of DNA segments (n=19) found in these 16 DNA fragments (separated before with DGGE) corresponded to *H. pylori* (n=8), as well to *H. rodentium* (n=4), *H. bilis* (n=3) and *H. hepaticus* (n=4) (fig. 3).

**Table 3. Sequencing of PCR products obtained after amplification of homogenates of stomach mucosa scraps (antral part)**

| Inoculates* | PCR products | *Helicobacter* spp. |
|---|---|---|
| *H. pylori* + PBS | 1. | *H. pylori* |
| (Group III) | 2. | *H. pylori* |
| | 3. | *H. pylori* |
| | 4. | *H. pylori* |
| | 5. | *H. pylori, H. bilis* |
| | 6. | *H. pylori* |
| | 7. | *H. pylori, H. bilis* |
| | 8. | *H. pylori* |
| *H. pylori* + AKG | 1. | *H. rodentium, H. bilis* |
| (Group III B) | 2. | *H. hepaticus* |
| | 3. | *H. rodentium* |
| | 4. | *H. hepaticus* |
| | 5. | *H. rodentium* |
| PBS + AKG | 1. | *H. hepaticus* |
| (Group IV B) | 2. | *H. hepaticus* |
| | 3. | *H. rodentium* |

| | | |
|---|---|---|
| */ according to the scheme in fig. 2. Animals exclusively infected with *H. pylori* (*H. pylori* + PBS), infected with *H. pylori* with following inoculation of salts of alpha-glutaric acid (*H. pylori* + AKG) and non infected, inoculated with salts of alpha-glutaric acid (PBS + AKG). | | |

As it follows from above Table 3. DNA of two *Helicobacter* species: *H. pylori* and *H. bilis* as well as *H. rodentium* and *H. bilis* were detected in 3 different mice. In stomach samples of 2 mice from group III (*H. pylori* + PBS) *H. pylori* and *H. bilis* was found. In stomach samples of mouse from group III B (*H. pylori* + salts of alpha-ketoglutaric acid) *H. rodentium* i *H. bilis* was detected (Table 3). Afterwards DNA of *H. hepaticus* was identified in 4 animals, two mice form group III B (*H. pylori* + salts of alpha-ketoglutaric acid), and in two mice inoculated with PBS + salts of alpha-ketoglutaric acid (Group IV B) (Table 3).

DNA of *H. bilis* did not appear separately in any PCR products, whereas DNA of *H. rodentium* was present in 3 samples without sequences accompanying, and in one sample together with DNA of *H. bilis* (fig. 3. Table 3).

In summary, number of colonies (mean ± SD) isolated from the antral part of the stomach mucosa of mice infected exclusively with *H. pylori* (Group III) was 4.3 x 10² ± 5.0 x 10¹ whereas after additional intragastric treatment of mice with salts of alpha-ketoglutaric acid (Grupa III B) no *H. pylori* colony was cultured (Table 2). Three times, during 3 consecutive days, intragastric induction of salts of alpha-ketoglutaric acid, which has been started after 8 days interval from the last infective dose of *H. pylori* bacteria for the mouse, caused total inhibitory effect on bacterial colonisation and fully eradication of *H. pylori* bacteria from gastric mucosa.

Moreover, during the time of experiment the composition of stomach urolytic microflora has been changed and in mice infected *H. pylori* (Group III) DNA of *H. bilis* was identified as well, whereas in mice, following inoculation with salts of alpha-ketoglutaric acid (Group III B) DNA of *H. rodentium, H. bilis, H. hepaticus* exclusively was found and in control mice treated with salts of alpha-ketoglutaric acid (Group IV B) DNA of *H. hepaticus* and *H. rodentium* was identified.

### Example 3.

Aqueous solutions of calcium and sodium salt of alpha-ketoglutaric acid in asmixture or separately were prepared and used as an additive for diary products and beverages.

| | |
|---|---|
| Salt of alpha-ketoglutaric acid | 0.001 g - 0.2 g |
| Glucose | 20 g |
| Water | up to 100 g |

Therapeutically and /or prophylactically effective amount is from 0.001g up to 0.2 g/kg bodyweight in day dose.

Using model animals (mice) and with the participation of volunteers it has been demonstrated that the intragastrical or oral administration of sodium and/or calcium alpha-ketoglutarate in the form of an aqueous solution, a prophylactic activity, alleviation of an infection course and also diminishing of colonisation of gastrointestinal tract by *H. pylori* are achieved.

The presented examples illustrate, how ureolytic bacteria - in this case *H. pylori -* compete against host organism cells for access to substrate, i.e. urea. Intragastric introduction of salts of alpha-ketogluteric acid facilitated the use of urea decomposed to ammonia by ureolytic bacteria for the needs of macroorganism, i.e. in synthesis of glutamate with alpha-ketoglutarate as one of the agents. In spite of the activity of bacterial urease, the acidic pH of the gastric environment was maintained preventing formation of a micro-niche for *H. pylori.* Thus, the colonisation of mucous membrane of macroorganisms by ureolytic bacteria was hindered and stopped. Use of alpha-ketoglutarate also prevents infections caused by ureolytic bacteria.

## Claims

1. Use of alpha-ketoglutarate for manufacturing of medical preparation for prophylaxis or treatment of undesired medical conditions related with the presence and/or activity of ureolytic bacteria in vertebrate, including a human being, mammal, bird, amphibian and fish, in formulations adjusted to administration to the occurrence site of targeted ureolytic bacteria.

2. The use according to claim 1, wherein the undesired medical conditions is a condition related with the presence and/or activity in the gastrointestinal tract, respiratory and/or urogenital system of ureolytic bacteria from the group including *Helicobacter pylori,* bacterial strains from the *Brucella* genus, urease-positive bacteria such as *Ureaplasma urealyticum* and other alkalophilic bacteria, such as for instance *Bacillus pasteurii*, urease-producing *Yersinia enterocolica* rods, ureolytic bacteria that participate in the formation of biofilm and mineralisation of deposits on catheters and other medical equipment, ureolytic bacteria causing infections of the mucous membrane in the oral cavity, gingival diseases, dental caries, causing formation of tartar, ureolytic bacteria responsible for formation of infectious stones in course of urinary system infections of the genii: *Proteus, Ureaplasma, Klebsiella, Pseudomonas, Staphylococcus, Providencia, Corynebacterium*, in particular *P. mirabilis,* and mycoplasmas causing genital tract - in particular its lower part infections, *Mycoplasma hominis* and *U. urealyticum.*

3. The use according to claim 1 or 2, wherein the preparation for preventing *H. pylori* colonisation and results thereof, in particular diseases such as stomach and duodenal ulcers, gastric lymphomas, chronic atrophic gastritis with intestines metaplasia and gastric cancer is manufactured.

4. The use according to claim 1 or 2, wherein the preparation for regulation of ureolytic intestinal flora, stabilising a systemic integrity and to be used to balance intestinal flora after infectious and cachectic diseases is manufactured.

5. The use according to claim 1 or 2, wherein the preparation for regulation of ureolytic flora in the oral cavity, reducing formation of tartar and inhibiting dental caries is manufactured in form of a chewing gum or a tooth paste.

6. The use according to claim 1 or 2, wherein the preparation for inhibition of passage of pathogenic ureolytic bacteria through stomach is manufactured.

7. The use according to claim 1 or 2, wherein the preparation preventing formation of deposits and infectious stones in urinary system is manufactured.

8. The use according to claim 1 or 2, wherein the preparation for inhibition of growth of ureolytic bacteria, in particular *Ureaplasma* and other mycoplasmas causing infections in fish is manufactured.

9. The use according to claim 8, wherein the preparation to be used in prophylaxis of gill inflammation in carp and carp fry, and other fresh water and sea fish caused by certain ureolytic bacteria is manufactured.

10. The use according to claim 1 or 2, wherein the preparation for reduction the formation of biofilm and mineralisation of deposits on catheters and other medical equipment is manufactured.

11. The use according to one of claims 1 to 10, wherein the preparation comprising alpha-ketoglutarate in quantities adjusted to administration in therapeutically effective dosage, in particular in the dosage from 0.001 to 0.2 g/kg of body mass/day is manufactured.

12. The use according to one of claims 1 to 11, wherein the preparation is in formulation for oral administration, in the form of infusions, irrigation liquids, intravaginal tablets and in other formulations adjusted to administration route.

13. Alpha-ketoglutarate for use as antibacterial agent effective against ureolytic bacteria when administered to the occurrence site of targeted ureolytic bacteria in vertebrate, including a human being, mammal, bird, amphibian and fish.

14. A medical preparation for use in prophylaxis and treatment of undesired medical conditions related with the presence and/or activity of ureolytic bacteria in vertebrate, including a human being, mammal, bird, amphibian and fish, in formulations adjusted to administration to the occurrence site of targeted ureolytic bacteria, manufactured in accordance with anyone of the claims 1 to 12.

15. Use of alpha-ketoglutarate for manufacturing of dietary supplement preventing undesired medical conditions related with the presence and/or activity of ureolytic bacteria in vertebrate, including a human being, mammal, bird, amphibian and fish.

16. Use of alpha-ketoglutarate for manufacturing of medicated food product preventing undesired medical conditions related with the presence and/or activity of ureolytic bacteria in vertebrate, including a human being, mammal, bird, amphibian and fish.

17. Use of alpha-ketoglutarate for manufacturing of food/feed additive, acting prophilactically, preventing colonisation by *H. pylori* of vertebrate, including a human being, mammal, bird, amphibian and fish.

## Patentansprüche

1. Verwendung des alpha-Ketoglutarates zur Herstellung eines Heilmittels zur Prophylaxe bzw. Behandlung unerwünschter medizinischer Zustände, verursacht durch das Auftreten und/oder die Aktivität ureolytischer Bakterien bei Wirbeltieren, darunter bei Menschen, Säugetieren, Vögeln, Amphibien und Fischen, in einer Zusammensetzung, die der Verabreichungsform in den Bereichen, wo die zu bekämpfenden ureolytischen Bakterien auftreten, angepasst ist.

2. Verwendung nach Anspruch 1, wo ein unerwünschter medizinischer Zuständ ein Zustand im Verdauungssystem, in den Atemwegen und/oder im Urogenitalsystem ist, verursacht durch das Auftreten und/oder die Aktivität folgender Bakterien: ureolytische Bakterien der Gruppe *Helicobacter pylori,* Bakterienstämme der Gattung *Brucella,* Urease-positive Bakterien wie *Ureaplasma urealyticum* und andere alkaliphilen Bakterien wie zum Beispiel *Bacillus pasteurii*, Ureaseproduzierende *Yersinia enterencolica*, ureolytische Bakterien, die bei Biofilmbildung und Mineralisierung von Ablagerungen an Kathetern und anderer medizinischen Ausrüstung mitwirken, ureolytische Bakterien, die Mundschleimhautinfektionen, Zahnfleischentzündungen, Karies, Zahnsteinbildung verursachen, ureolytische Bakterien der Gattung *Proteus, Ureaplasma, Klebsiella, Pseudomonas, Staphylococcus, Providencia, Corynebacterium* und insbesondere *P. mirabilis,* die eine Infektionssteinbildung bei Infektion des Harnsystems verursachen, Mykoplasmen, die Genitaltraktinfektionen, insbesondere im unteren Abschnitt verursachen, sowie *Mycoplasma hominis* und *U. urealyticum*.

3. Verwendung nach Anspruch 1 oder 2, wo ein Bokämpfungsmittel gegen die Besiedlung durch die Bakterien *H. pylori* und gegen die Folgen der Besiedlung durch die *H. pylori,* insbesondere gegen solche Krankheiten, wie Magen- und Zwölffingerdarmgeschwüre, Magenlymphome, chronische atrophische Gastritis mit intensinaler Metaplasie und Magenkrebs, hergestellt wird.

4. Verwendung nach Anspruch 1 oder 2, wo ein Mittel zur Regulierung ureolytischer Darmmicrobiota, Stabilisierung systemischer Integrität und zum Ausgleich der Darmmicrobiota nach Infektions- und auszehrenden Krankheiten, hergestellt wird.

5. Verwendung nach Anspruch 1 oder 2, wo ein Mittel zur Regulierung ureolytischer Mundmicrobiota, Begrenzung der Zahnsteinbildung und Karieshemmung, in Form von Kaugummi oder Zahnpasta hergestellt wird.

6. Verwendung nach Anspruch 1 oder 2, wo ein Mittel zur Hemmung der Passage durch den Magen pathogener ureolytischer Bakterien, hergestellt wird.

7. Verwendung nach Anspruch 1 oder 2, wo ein Mittel zur Verhinderung der Bildung von Ablagerungen und Infektionssteinen im Harnsystem hergestellt wird.

8. Verwendung nach Anspruch 1 oder 2, wo ein Mittel zur Hemmung des Wachstums der ureolytischen Bakterien, insbesondere der *Ureaplasma* und anderer Mycoplasmen, die Infektionen bei Fischen verursachen, hergestellt wird.

9. Verwendung nach Anspruch 8, wo ein Mittel zur Anwendung bei der Prophylaxe der Kiemenentzündungen bei Karpfen und Karpfenbrut sowie bei anderen Süßwasser- und Seefischen, die von bestimmten ureolytischen Bakterien verursacht werden, hergestellt wird.

10. Verwendung nach Anspruch 1 oder 2, wo ein Mittel zur Begrenzung von Biofilmbildung und Mineralisierung der Ablagerungen auf Kathetern und auf einer anderen medizinischen Ausrüstung, hergestellt wird.

11. Verwendung nach einem der Ansprüche 1 bis 10, wo ein Mittel hergestellt wird, das das alpha-Ketoglutarat in einer Menge enthält, die der therapeutisch wirksamen Dosis, insbesondere von 0,001 bis 0,2 g/kg des Körpergewichts/Tag, angepasst ist.

12. Verwendung nach einem der Ansprüche 1 bis 11, wo ein Mittel zur peroralen Anwendung als Infusionslösungen, Bewässerungsflüssigkeiten, Vaginalzäpfchen oder in einer anderen Zusammensetzung, die der Verabreichungsform angepasst ist, hergestellt wird.

13. Das alpha-Ketoglutarat zur Anwendung als antabakterielles Mittel gegen die ureolytischen Bakterien bei einer Therapie in den Bereichen, wo die zu bekämpfenden ureolytischen Bakterien bei Wirbeltieren, darunter bei Menschen, Säugtieren, Vögeln, Amphibien und Fischen auftreten.

14. Ein Arzneimittel zur Anwendung bei der Prophylaxe und Heilung unerwünschter medizinischer Zustände, die durch das Auftreten oder/und die Aktivität ureolytischer Bakterien bei Wirbeltieren, darunter bei Menschen, Säugtieren, Vögeln, Amphibien und Fischen verursacht sind, dessen Zusammensetzung der Verabreichungsform in den Bereichen, wo die zu bekämpfenden ureolytischen Bakterien auftreten, angepasst ist.

15. Verwendung des alpha-Ketoglutarates zur Herstellung eines Nahrungsergänzungsmittels gegen unerwünschte medizinische Zustände, die durch das Auftreten oder/und die Aktivität ureolytischer Bakterien bei Wirbeltieren, darunter bei Menschen, Säugetieren, Vögeln, Amphibien und Fischen verursacht sind.

16. Verwendung des alpha-Ketoglutarates zur Herstellung eines medizinischen Nahrungsmittels zur Verhinderung unerwünschter medizinischer Zustände, die durch das Auftreten oder/und die Aktivität ureolytischer Bakterien bei Wirbeltieren, darunter bei Menschen, Säugetieren, Vögeln, Amphibie und Fischen verursacht sind.

17. Verwendung des alpha-Ketoglutarates zur Herstellung eines Lebensmittel/Futterzusatzstoffes zur Prophylaxe gegen die Besiedelung durch die Bakterien *H. pylori* bei Wirbeltieren, darunter bei Menschen, Säugetieren, Vögeln, Amphibien und Fischen.

## Revendications

1. L'usage de l'alpha-cétoglutarate à la production d'un remède curatif pour la prophylaxie ou le traitement des événements médicaux indésirables liés à la présence et/ou de l'activité des bactéries uréolytiques chez le vertébré, y compris l'homme, le mammifère, l'oiseau, le batracien et le poisson, dans les formulations adaptées à l'administration à l'endroit d'apparition des bactéries uréolytiques combattues.

2. L'usage selon la revendication 1, dans laquelle l'événement médical indésirable est celui lié à la présence et/ou l'activité dans les systèmes digestif, respiratoire et/ou urogénital des bactéries uréolytiques du groupe comprenant *Helicobacter pylori,* les souches de genre *Brucella,* les bactéries uréase positives telles que *Ureaplasma urealyticum* et autres bactéries alcalophiliques (alcalophiles), telles que, à titre d'exemple, *Bacillus pasteurii*, produisant l'uréase de la bacille *Yersinia enterocolica,* les bactéries uréolytiques qui prennent part dans la formation du biofilm et la minéralisation des concrétions sur les cathéters et autre équipement médical, les bactéries uréolytiques causant les infections de la muqueuse buccale, les douleurs gingivales, la carie dentaire et causant la formation du tartre dentaire, les bactéries uréolytiques responsables de la formation des calculs infectueux au cours de l'infection du système urinaire - de genre *Proteus, Ureaplasma, Klebsiella, Pseudomonas, Staphylococcus, Providencia, Corynebacterium*, notamment *P. mirabilis* et les mycoplasmes causant l'infection des voies génitales - en particulier les infections de leur section inférieure, *Mycoplasma hominis* et *U. urealyticum*.

3. L'usage selon la revendication 1 ou 2, dans laquelle est produit le moyen préventif contre la colonisation *H. pylori* et ses effets, notamment contre les maladies telles que l'ulcéreuse de l'estomac et du duodénum, les lymphomes de l'estomac, la gastrite atropique chronique avec la métaplasie intestinale ainsi que le cancer de l'estomac.

4. L'usage selon la revendication 1 ou 2, dans laquelle est produit le moyen pour la régulation uréolytique du microbiote intestinal, stabilisant l'intégralité systémique et à l'usage pour balancer le microbiote intestinal suite aux maladies infectieuses et cachectiques.

5. L'usage selon la revendication 1 ou 2, dans laquelle est produit le moyen pour la régulation uréolytique de le microbiote buccale, réduisant la formation du tartre dentaire et assurant l'inhibition la carie dentaire, sous forme de la gomme à mâcher ou la dentifrice.

6. L'usage selon la revendication 1 ou 2, dans laqulle est produit le moyen pour l'inhibition du passage des bactéries uréolytiques pathogènes à travers l'estomac.

7. L'usage selon la revendication 1 ou 2, dans laquelle est produit le moyen préventif contre la formation des concrétions et calculs infectieux dans le système urinaire.

8. L'usage selon la revendication 1 ou 2, dans laquelle est produit le moyen pour l'inhibition d'accroissement des bactéries uréolytiques, notamment *Ureaplasma* et d'autres mycoplasmes causant les infections chez les poissons.

9. L'usage selon la revendication 8, dans laquelle est produit le moyen pour l'administration en prophylaxie contre l'inflammation des branchies de la carpe et de son alevin ainsi que chez d'autres poissons d'eau douce et de mer, causée par certaines bactéries uréolytiques.

10. L'usage selon la revendication 1 ou 2, dans laquelle est produit le moyen pour réduire la formation du biofilm et la minéralisation des concrétions sur les cathéters et autre équipement médical.

11. L'usage selon de la revendication quelconque de 1 à 10, dans laquelle est produit le moyen contenant l'alpha-cétoglutarate en quantité adaptée à l'administration en dose thérapeutiquement efficace, en particulier en dose de 0,001 jusquà 0,2 g/l kg de masse corporelle/1 jour.

12. L'usage selon de la revendication quelconque de 1 à 11, dans laquelle est produit le moyen pour administrer par voie orale, sous forme de lavements, liquides d'irigation, comprimés intravaginaux ou en autres formulations adaptées à la voie d'administration.

13. L'alpha-cétoglutarate à administrer comme le moyen antibactérien et efficace contre les bactéries uréolytiques, quand il est administré à l'endroit d'apparition des bactéries combattues chez le vertébré, y compris l'homme, le mammifère, l'oiseau, le batracien et le poisson.

14. Le moyen curatif à administrer en prophylaxie et le traitement des événements médicaux indésirables liés à la présence et/ou l'activité des bactéries uréolytiques chez le vertébré, y compris l'homme, le mammifère, l'oiseau, le batracien et le poisson en formulations adaptées à l'administration à l'endroit d'apparition des bactéries uréolytiques combattues, produit conformément à la revendication quelconque de 1 à 12.

15. L'usage de l'alpha-cétoglutarate pour la production du supplément de la diète, préventif des événements médicaux indésirables, liés à la présence et/ou l'activité des bactéries uréolytiques chez le vertébré, y compris l'homme, le mammifère, l'oiseau, le batracien et le poisson.

16. L'usage de l'alpha-cétoglutarate pour la production du moyen alimentaire d'affectation médicale spéciale, préventif des événements médicaux indésirables liés à la présence et/ou l'activité des bactéries uréolytiques chez le vertébré, y compris l'homme, le mammifère, l'oiseau, le batracien et le poisson.

17. L'usage de l'alpha-cétoglutarate pour la production de l'additif à la nourriture/fourrage prophylactique et préventif de la colonisation par *H. pylori* du vertébré, y compris l'homme, du mammifère, de l'oiseau, du batracien et du poisson
